Europäisches Patentamt

European Patent Office

Office européen des brevets

(19)

(11) Publication number: **0 286 456**
**A1**

(12) **EUROPEAN PATENT APPLICATION**

(21) Application number: **88400121.5**

(22) Date of filing: **20.01.88**

(51) Int. Cl.⁴: **A 61 B 5/02**

(30) Priority: **08.04.87 ES 8701264**

(43) Date of publication of application:
**12.10.88 Bulletin 88/41**

(84) Designated Contracting States:
**AT BE CH DE FR GB GR IT LI LU NL SE**

(71) Applicant: **TAKIO, S.A.**
**2, L'Encuny Street**
**E-08004 Barcelona (ES)**

(72) Inventor: **Fernández Belmonte, Asensio**
**36, Virgen del Pilar, Str.**
**E-08320 El Masnou (Barcelona) (ES)**

(74) Representative: **Tony-Durand, Serge**
**Cabinet Tony-Durand 77, rue Boissière**
**F-75116 Paris (FR)**

(54) **An electronic apparatus for medical diagnosis.**

(57) An electric apparatus for medical diagnosis is provided, placed inside a case and including a central module capable of controlling the electrocardiograms, different pulmonary functions, phono-mecano-cardiographies and auscultations, blood pressure and liminal audiometry, having keys and inputs required for introducing signals hot only directly but also over telephone lines, as well as outputs for knowing and recording said information as well as transmission thereof to another center.

Fig. 2

EP 0 286 456 A1

**Description**

## AN ELECTRONIC APPARATUS FOR MEDICAL DIAGNOSIS

The present invention relates to an electronic apparatus for medical diagnosis, having as fundamental specific characteristic the fact that it associates in a single apparatus different devices for carrying out a complete check up.

Another advantage of the apparatus of the invention with respect to the apparatus existing at the present time on the market, flows from the fact that the data cannot only be obtained during the consultation with the doctor but also from the fact that the patient may also obtain them in a distant place, such for example as his own home, the equipment being provided for receiving signals over a telephone channel, this is why it is necessary to have available therefor a terminal for storing said information and then transmitting it, said terminal being also able to be handed over to the patient to be unloaded directly in the apparatus of the invention, said intermediate equipment acting as decoder and means for storing the data obtained, which may then also be retransmitted to a more important center of diagnosis if the doctor thinks it necessary.

It is then an apparatus of advanced concept and compact design which allows all the advantages of telematics to be introduced in the medical field.

The apparatus of the invention forms a total revolution in the field of devices at present existing on the market, for it incorporates in a single compact element of very small volume the majority of functions which the doctor must know concerning the patient, enormously facilitating the acquisition thereof, with the additional above mentioned advantages.

The functions provided by the apparatus described are the following:

A. Electrocardiogram for which twelve tappings are provided which are normally used and usually known under the abbreviations:

DI
DII
DIII
aVL
aVR
aVF

as well as the six precordial tappings.

B. Analysis of the pulmonary function for which a flow rate transducer is provided for calculating the following parameters of forced expiration:

Forced vital volume
Expired volume in the first second
Meso-respiratory flow rate
Duration of expiration
Maximum expired rate
Rate at the moment when about 25% of the volume to be expired is lacking
Rate at the time when about 50% of the volume to be expired is lacking
Rate at the time when about 75% of the volume to be expired is lacking.

In addition to this data, the apparatus records the curves:
- volume/time
- flow rate/time.

The parameters calculated are compared with the statistic data for knowing the differences therebetween.

As additional advantage, as mentioned above, it is possible to carry out this function not only with the patient close to the equipment, in local operation, but also operating through the telephone.

In the case where the recordings are carried out through a telephone channel, the equipment has two transducers which transform the electric signals of the electrocardiogram or of the flow rate of the air expired into frequency signals included in the telephone range. In this situation, the apparatus of the invention receives the telephone signals through a microphone and it transforms them into graphs and described numerical values.

C. Phono-mecano-cardiography and auscultation. Ausculation allows the cardiac sounds picked up by means of a microphone to be recorded by amplifying them and injecting them into a stethoscope so that the doctor may go ahead with an auscultation with a sound intensity greater than that corresponding to the use of a phonic apparatus.

The phono-mecano-cardiography consists in recording these previously filtered cardiac sounds, this is why filters are provided having the following characteristics:
- Low band, low pass filter with cut off frequency of 100 Hertz, with an attenuation from a frequency of 40 dB/decade.
- Medium band, band pass filter between 100 and 200 hertz, the attenuations from this frequency are 40 dB/decade,
- High band, filter for 200 to 600 Hertz band, having similar characteristics to the preceding ones.

Recording for the low frequencies is a line which reproduces the signal received. In the high frequency range, the envelope of the signal is recorded.

D. Blood pressure. For measuring the pressure, the equipment is fitted with an inflatable sleeve with a microphone.

The procedure for measuring the pressure is the following:
- A pressure is applied on the sleeve until the sounds produced by the passage of the blood in the artery disappear.
- This pressure is reduced at the rate of 3mm Hg/second.
- At the moment when the first arterial noise appears the blood pressure is recorded as maximum pressure.
- At the moment when the noises caused by narrowing of the artery disappear, the minimum blood pressure is recorded.

E. Liminal audiometry. The procedure used by the equipment for determining the minimum threshold of audibility is the following.

Through earphones an audible power of 40 dB is injected (the scale of measurement is the clinical scale) and it is reduced until the patient subjected to the test ceases to hear the sound produced, this analysis being repeated at different selected sequences for determining the different audibility thresholds.

All and each of these functions are incorporated in a single compact device, having the necessary inputs so that, after being disposed on the patient, the apparatus may send the signals indispensable for controlling said functions, and a keyboard as well as a central microprocessor module which processes the information, making transmission thereof possible to the outside through the necessary outputs, such as printers, displays or for sending over a telephone line.

To facilitate the explanations, reference will be made to the accompanying drawings in which one embodiment of an electronic medical diagnosis apparatus has been shown by way of illustrative but non limitative example, in accordance with the principles of the claims.

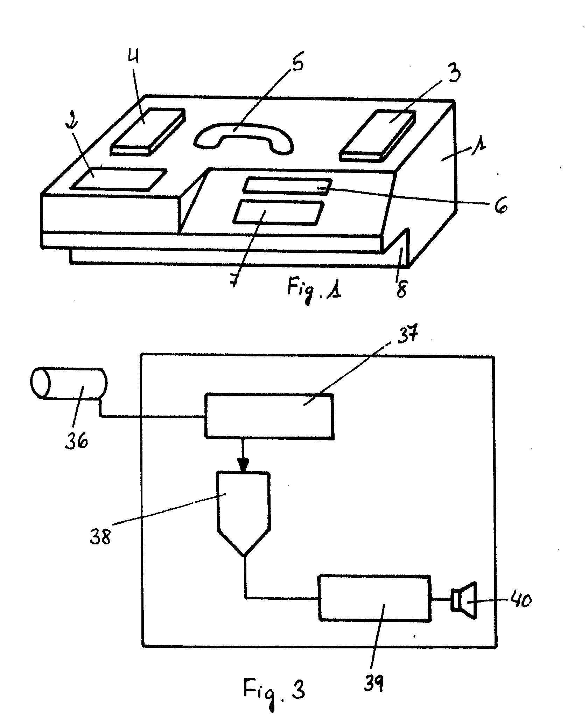

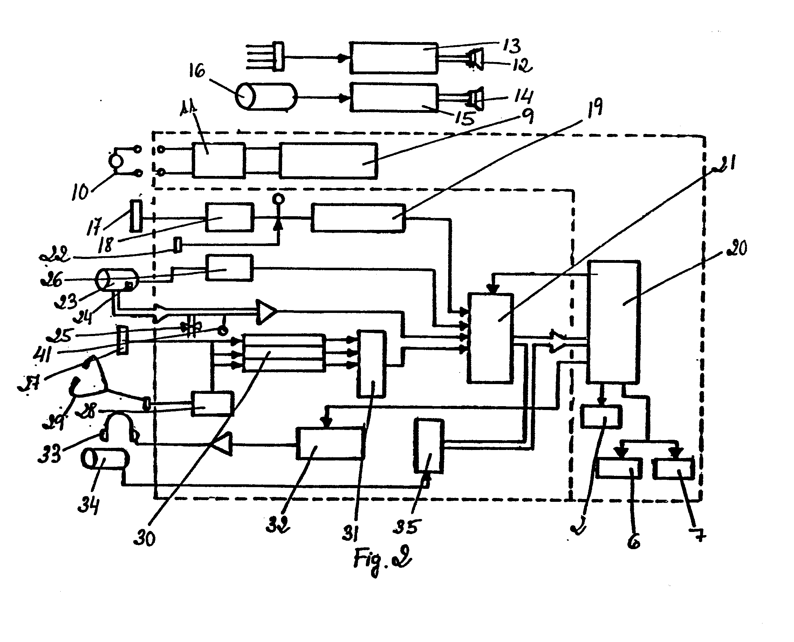

In the drawings, Figure 1 shows a perspective view of the eternal appearance of the apparatus of the invention; and

Figure 2 shows a block diagram which allows to be accurately known of the different functions forming the apparatus of the invention.

As is clear from the drawings, the frame 1 as a whole of the apparatus of the invention is provided with a digital and graphic printer 2, an element 3 for sending the electrocardiogram by telephone, another element 4 for transmitting the air flow rate signals by telephone, an earphone 5 for receiving the telephone signals, a liquid crystal display 6, a keyboard 7 and, inside the unit, the central module 8.

For a better understanding of the fundamental internal elements of the device, a block diagram has been drawn up in which the different elements forming it are shown and numbered.

Thus, the DC power supply source for apparatus 9 is connected to the mains 10 through a filter 11.

Insofar as the external element 3 is concerned, it has a loudspeaker 12 for the telephone transmission, a converter 13 for converting the signals to the telephone frequency, whereas element 4, which allows transmission of airflow rate signals by telephone, is also formed by another loudspeaker 14, by the corresponding pulse/telephone frequency converter 15 and transducer 16.

The functions possessed by the equipment are carried out as described below.

For controlling the electrocardiograms, element 3 receives the electric signals from the patient over cables which are connected permanently to the terminals thereof, whereas the cardiogram is produced and there is a fifth terminal which the doctor must place at certain predetermined points on the chest of the patient for carrying out so called precordial recordings.

The electric powers are transformed by a voltage/frequency converter into signals with a frequency range included in the audible range, with the following transformation characteristics:

- signal potential volts - equivalent to 1900Hz
- signal potential 1mV - equivalent to 2000Hz
- signal potential 1mV - equivalent to 1800Hz.

If it is desired to transmit the electrocardiogram by telephone, the above described loudspeaker 12 is used which allows the sound produced to be transmitted to the telephone network.

The procedure for telephone reception on the central module 8 is carried out using the following procedure: the telephone signal picked up by microphone 17, suitably filtered at 18, is fed to a frequency/voltage converter 29 having the following characteristics:

- signal of 1900 Hz corresponds to an output of 0 V,
- signal of 2000 Hz corresponds to an output of 1mV
- signal of 1800Hz corresponds to an output of 1mV.

The microprocessor 20 reads these analog values by means of an A/D converter 21 and it controls the printer which prints the dots.

In the case of receiving an electrocardiogram by the local procedure, the signal produced, instead of being sent by telephone, is connected directly to 22 and, from there, the procedure continues in identical fashion.

The pressure is measured in the following way.

Once the patient has passed his arm into sleeve 23, the microphone 24 which is in this sleeve remains positioned on the humeral artery and, using the compresser 41, air is injected at that time into the sleeve until a pressure of 180mm column of mercury is obtained.

Then the signal from the microphone is read so as to detect the Korotcoff noises, their disappearance indicating that the maximum blood pressure of the patient is less than 180mm Hg, whereas the opposite case, the pressure in the sleeve is increased up to 250mm of Hg.

In this case, valve 25 is opened which causes a pressure drop in the sleeve of the order of 3mm of mg each second, and the pressure is noted which coincides with the first and the last Korotcoff noise, and which corresponds to the maximum and minimum blood pressure of the patient.

Microprocessor 20, to which the signal duly filtered at 26 is fed, controls the whole procedure and reads the values of the pressure, of the amplitude of the Korotcoff noises, by means of the A/D converter 21.

For carrying out the phono-mecano cardiography and the auscultation, microphone 27 is used which is the one which records the cardiac noises.

Amplifier 28 amplifies the signal from the microphone and energizes a loudspeaker housed in a hermetic case. During this time, the doctor's stethoscope 29 is connected to this case so that the vibrations produced by the loudspeaker are fed directly thereto, so that the low frequency vibrations are transmitted more efficiently to the doctor's ear.

Phono-mecano-cardiography consists in filtering, by means of the 3 filters 30, the sounds picked up by the microphone and in printing the recording thereof by means of the dot printer 2.

The procedure followed by putting this function into practice is the following:

Depending on the keys pressed, the microprocessor 20 selects one of the filters 30. Through the dot detector 31, the maximum value and minimum value detector reads every 5 milliseconds the maximum value and the minimum value of the filtered signal and imprints on printer 2 a vertical segment corresponding to these two values.

In the case of low frequencies, the printer follows the signal received, in the high frequencies the procedure corresponds to the envelope of the filtered signal.

Insofar as liminal audiometry is concerned, the operating mode is as follows:

Microprocessor 20 may control on the signal generator 32 the frequency of the power of the signal produced, while feeding it into the audiometry earphones 33.

The range of frequencies which it is possible to produce extends from 32Hz up to 4000Hz and the power measured in clinical decibels goes from 0 to 80 dB.

For carrying out an audiometry test, the frequencies of the clinical powers are selected manually or else the microprocessor 20 may initiate a sequence of pre-established frequencies and powers.

Insofar as a pulmonary function is concerned, it is possible to act in two separate ways.

In the case of operation in a local system, transducer 34 gives 150 pulses/liter which are counted by means of the pulse counter 35, the microprocessor 20 reading every 5 milliseconds the value of the counter, which thus allows the value of the flow rate and the expired volume to be known and, from these data, it is possible to recompose the volume/time curves and to calculate mathematically all the values which are indicated subsequently.

In the case of a telephone transmission and as shown in Figure 3 the expirometry module begins in the same way with transducer 36 whose pulses are counted by means of an 8 bit counter 37 which is reset on arriving at 256 and which then begins to count again. The sound produced is proportional to the value of the counter, which is obtained by using a D/A converter 38 and a voltage/frequency converter 39, which finally sends the signal to the loudspeaker 40.

The descriptions given in the above paragraphs demonstrate, by using for that the numerical references which appear in the drawings, the different functions which it is possible to control with the electronic medical control apparatus of the invention, while demonstrating the existence, on this latter, of the inputs and outputs required for introducing it into a large telematics network, which begins on the one hand with the patient who may be near or far from the apparatus using, in this latter case, a telephone transmission extending as far as the patient or else by sending the data stored on an adequate carrier for transmission thereof after processing by telephone also, to the higher level service.

All that does not affect, does not alter, does not change or modify the essence of the apparatus described will be considered as a variant within the scope of the present invention.

## Claims

1. Electronic apparatus for medical diagnosis characterized by the fact that in a single compact carcase there is incorporated a central module capable of controlling electro-cardiograms, different pulmonary functions, phono-mecano-cardiographies, auscultations, blood pressure measurement and liminal audiometry, including a keyboard and the inputs required for introducing the signals, either directly, or through a telephone line, as well as the output required for the knowledge of said information, such as printers, liquid crystal displays and devices for connecting and transmitting to a more important center of diagnosis the telephone signals coming from the electrocardiograms or expirometries.

2. Electronic apparatus for medical diagnosis according to claim 1, characterized by the fact that by providing telephone access to data, it makes possible acquisition thereof by the patient outside the doctor's consulting room and also at different times in the day, for subsequent telephone transmission to said apparatus at the opportune moment.

3. Electronic apparatus for medical diagnosis according to claim 1, characterized by the fact that for controlling the electrocardiograms, the electric signals from the patient are received through the telephone, at a microphone whose filtered signal is fed to a frequency and voltage converter which reads the analog values through an A/D converter and which controls the printer on which the corresponding dots are printed, whereas, in the case of an electrocardiogram carried out close to the apparatus, the signal produced is connected directly to an element which introduces it in the procedure from which there is coincidence with the above described method.

4. Electronic apparatus for medical diagnosis according to claim 1, characterized by the fact that for carrying out the blood pressure measurement it is provided with a sleeve with incorporated microphone in which air is injected by means of a compressor up to a given pressure, detected the minimum and maximum values being in a microprocessor which reads the values of the pressure through an A/D converter.

5. Electronic apparatus for medical diagnosis according to claim 1, characterized by the fact that for carrying out phono-mecano-cardiographies and auscultations, the cardiac noises are recorded by means of a microphone having an amplifier which increases the signal and which energizes a loudspeaker closed in a hermetic case, to which the doctor's stethoscope is linked which thus picks up the low frequency vibrations with greater ease for then filtering, by means of three separate filters, the sounds picked up by the microphone, which are selected by the microprocessor with reading by means of a dot detector, every five seconds, of the maximum value and of the minimum value of the filtered signal, while printing on the printer a vertical segment corresponding to these values.

6. Electronic apparatus for medical diagnosis according to claim 1, chararacterized by the fact that for carrying out liminal audiometry, the microprocessor may control on the generator the range of signals, the frequency and the power of the signal produced, while feeding it into the audiometry earphones and making possible the generation of ranges of frequencies between 32Hz and 4000Hz and with a mean power in clinical decibels between 0 and 80, the microprocessor being able to allow manual section of the clinical frequencies and powers or initiating a pre-established sequence thereof.

7. Electronic apparatus for medical diagnosis according to claim 1, characterized by the fact that for carrying out expirometry in a local system, a transducer is provided which gives 150 pulses/liter, which are counted by means of a pulse counter whose count is read every 5 milliseconds by the microprocessor which thus knows the value of the flow rate and the expired volume, from which it calculates the volume/time curve and, after mathematical calculation, different parameters such as the forced vital volume, the expired volume at the first second, the meso-expiratory flow rates, the expiration time, the maximum expired rate as well as the rates at different times of the expired volume, whereas in the case of transmission of the expirometry to the apparatus by way of telephone, another transducer is provided whose pulses are counted by means of an 8 bit counter which is reset on reaching 256 and which begins the count again, the sound produced being proportional to the value of the counter, which result is obtained because of the existence of a D/A converter and a voltage/frequency converter.

Fig. 1

Fig. 3

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | US-A-3 857 383 (I.H. SOMMERFELD et al.) <br> * Abstract; column 2, line 43 - column 3, line 60; figure 1 * <br> --- | 1 | A 61 B 5/02 |
| Y | US-A-4 608 994 (L. OZAWA et al.) <br> * Whole document * | 1 | |
| A | | 2,4 | |
| A | JOURNAL OF ELECTRONICS AND ENGINEERING, no. 110, February 1976, pages 20-22, Tokyo, JP; H. KUBOTA: "Intensive patient monitoring systems save time and labor in hospitals" <br> * Whole article * <br> --- | 1 | |
| A | US-A-3 882 277 (D. DEPEDRO et al.) <br> * Abstract; column 5, line 19 - column 6, line 11; figure 1 * <br> --- | 1,3 | |
| A | PROCEEDINGS OF THE IEEE, vol. 65, no. 5, May 1977, pages 714-722; M. BERTRAND et al.: "Microprocessor application for numerical ECG encoding and transmission" <br> * Whole article * <br> --- | 3 | TECHNICAL FIELDS SEARCHED (Int. Cl.4) <br><br> A 61 B |
| A | PATENT ABSTRACTS OF JAPAN, vol. 3, no. 135 (E-150), 10th November 1979, page 157 E 150; & JP-A-54 114 008 (NIPPON DENSHIN DENWA KOSHA) 05-09-1979 <br> * Abstract * <br> --- | 5 | |
| A | DE-C- 837 581 (W. HALSCHEIDT et al.) <br> * Whole document * <br> ---       -/- | 5 | |

The present search report has been drawn up for all claims

| Place of search | Date of completion of the search | Examiner |
|---|---|---|
| THE HAGUE | 08-07-1988 | FERRIGNO, A. |

EPO FORM 1503 03.82 (P0401)

European Patent Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 489 610 (M.J. SLAVIN)<br>* Whole document *<br>--- | 6 | |
| A | EP-A-0 196 396 (COSMED)<br>* Abstract; figure 1 *<br>----- | 7 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 08-07-1988 | FERRIGNO, A. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)